# EUROPEAN PATENT APPLICATION

(11) **EP 0 824 023 A1**
(43) Date of publication of application: **18.02.1998**
(21) Application number: 96112926.9
(22) Date of filing: 12.08.1996
(51) Int. Cl.: A61M 15/00

(54) **Respiratorial drug administration device**

(71) Applicant: Microflow Engineering SA, 2007 Neuchâtel (CH)
(72) Inventor: Hess, Joseph, 2022 Bevaix (CH)
(74) Representative: Patry, Didier Marcel Pierre

(57) **Abstract**

Device for the respiratorial administration of a drug to a patient, comprising :
- a flow sensor (16) for detecting an inspiratory flow of said patient;
- drug delivery means (10) for delivering an amount of said drug;
- control means (11) for causing the operation said drug delivery means, characterised in that said control means (11) are adapted to selectively operate said drug delivery means (10) such that the rate at which said drug is delivered during said inspiratorial flow is dependent upon the temporal value of said inspiratory flow.

## Description

The present invention relates generally to drug administration devices, and in particular to devices for administrating a drug to a patient by means of his or her respiratorial system. The invention is applicable in the medical field for the controlled administration of drugs, for the treatment of conditions such as asthma and diabetes, by nasal or oral inspiration. It will be convenient to herein after describe the invention in relation to that exemplary application, although it should be appreciated that the invention is not limited to the application. The invention can be used, for example, for a variety of systemic therapies involving drug formulations as solutions, suspensions or emulsions in ambulant treatment or hospital based therapy.

Various devices for the administration of a drug by inspiration are known. One type of commercially available device is a metered dose inhaler which delivers a fixed dosage of medication to a patient upon each activation of the device. This type of inhaler includes a canister containing a reservoir of a particular drug and propellant under pressure. The canister is inserted into a receptacle in a housing having a mouthpiece or nosepiece. The device may be manually operable by the patient, whereupon a fixed volume of drug is delivered into the mouthpiece as an aerosol mist. Simultaneously, the patient inhales through the mouthpiece to entrain the mist into his or her airway.

A problem with such metered dose inhalers is that the patient frequently activates the device either too early or too late during inspiratory flow to obtain the full benefit of the intended drug therapy, thus limiting the drug delivery efficiency of these devices.

Another problem with such pressurised metered dose inhalers is that the propellants, e.g. CFC's and HFA's, are thought to cause environmental problems and are or will be phased out for that reason.

Another problem of pressurised metered dose inhalers is the high speed with which the mist is propelled into the buccal cavity and the inconsistency of dosage over time, leading to differences in the delivered dose and actually inhaled dose, to variation in availability of the drug in the deep lung and to a lack of reproducibility of all these factors.

Another type of inhaler is a breath-actuated metered dose inhaler which operates to automatically provide a metered dose in response to the patient's inspiratory effort. The delivery of the drug in the form of an aerosol mist is triggered when the inspiratory effort crosses a fixed threshold.

Existing breath-actuated metered dose inhalers have not, however, entirely overcome the problem of timing the delivery of the drug during the patient's inspiration. Firstly, the inspiratory efforts of some patients may be insufficient to rise above the threshold at which the metered dose of drug is released. Secondly, even if an inspiration effort is be sufficient to release the drug dose, the inspiratory flow following release may not be sufficient to cause the aerosol medication to pass into the desired portion of the patient's airways.

Another problem with such metered dose inhalers in that they always provide a single, uniform dose of medication which is delivered at the time when the device is activated.

One known breath-actuated metered dose inhaler addresses these problems by releasing a number of metered doses of an aerosol compound at different firing points during the patient's inspiration. The firing points may be preprogrammed, or may be determined from a previous inspiratory flow. Whilst the delivery of the drug at the various firing points is still triggered when the inspiratory effort crosses a corresponding threshold, these thresholds are recursively lowered for later aspirations if the inspiratory flow was insufficient to trigger the release of the drug at all firing points.

Nevertheless, whilst using such a breath-actuated metered dose inhaler, a patient having a poor inspiration capability may be required to inspire one or more times before the drug-bearing mist is released, potentially compounding the emotional problem of an asthma patient. In addition, the firing points for a particular inspiratory flow are only determined from a previous inspiratory flow, and are not necessarily well matched to that particular inspiratory flow.

It can thus be seen that this type of inhaler does not adequately address the problem of the timing the delivery of the drug during the patient's inspiration. Moreover, such an inhaler is complicated for most patients to use.

Such an inhaler also suffers from the disadvantage that it is complicated to manufacture and program. It also requires the use of expensive and, in respect of its control circuitry, energy consuming components.

Another type of inhaler is a nebulizer in which the aerosol is produced as a jet by compressed air, sometimes using electronically controlled valves which are triggered by the patient's inhalation. The aerosol in nebulizing inhalers is sometimes generated by ultrasound whereby a cloud of particles is generated out of the liquid dose in a multidose reservoir, the non-respired fraction returning to that reservoir. A key problem of these nebulizing inhalers is the potentially poor hygienic conditions which exist in the nebulizer between therapy sessions or treatments.

Another type of inhaler is a dry powder inhaler where the drug is contained in a bulk or unit dose dry powder formulation. In the bulk type dry powder inhaler the individual dose is usually milled, shaved or otherwise removed from a solid bulk cylinder, sphere or other body containing the compressed drug formulation. The resulting cloud of fine powder particles is inhaled by the patient, using the patients inspiratory energy to transport the drug particles into his or her airway. To assist the delivery, especially for asthma therapy in patients with very low inspiratory capacity (for example, less than 15 litres/min), special ducting for turbulent inspiratory flow for particle transport, spring loading for dose projection or sometimes a motorised ventilator is used. Some of these solutions lead very often to the same breath and drug release timing coordination problems as previously described as well as to a lack of reproducibility as described above for pressurised metered dose inhalers. The complexity of such devices also increases their cost and decreases their user-friendliness.

In the unit dose dry powder inhalers the dry powder formulation is contained in individual doses, enclosed in blisters, capsules or other means which are cut open or otherwise opened abruptly or sometimes compressed to permit inhaling of the fine powder contents. The most relevant problem to dry powder inhalers, beside the similarities with the problems of pressurised metered dose inhalers and dry powder inhalers, is the difficulty of maintaining absolute dryness of the bulk or unit dose in a variety of climates and environments and to prevent the lumping of the fine particles, once released. Any lumping of course increases impactation of particles within the buccal cavity, reduces the availability of the drug in the deep lung and the efficacy of the treatment.

Additional to these problems, pressurised metered dose inhalers and dry powder inhalers are presently ill-equipped to assist their user with diagnostic information, provided directly or via a device interface. This leads to frequent over or underdosage which can be dangerous, especially for systemic respiratory therapies, such as insulin therapy via inhalers. Such diagnostic information is useful to inform the patient of the efficacy of each individual therapeutic dose, and also to enable to construct daily delivery profiles tailored to the patient's condition. The availability of time coordinated diagnostic reading and therapeutic treatment data also assists the physician in the follow-up of patients following systemic therapies, such as diabetics for example.

There remains, therefore, a need to improve the efficacy, simplicity and ease-of-use of such drug administration devices.

It is an object of the present invention to alleviate these problems through the provision of a respiratory drug administration device which provides efficient and accurate drug delivery during the inspiratory flow of a patient.

Another object to the invention is to provide a respiratory drug administration device which alleviates or overcomes the problems of known respiratory drug administration devices.

With this in mind, the present invention provides a device for the respiratorial administration of a drug to a patient, comprising a flow sensor for detecting an inspiratory flow of said patient, drug delivery means for delivering an amount of the drug, and control means for causing the operation the drug delivery means, characterised in that the control means are adapted to selectively operate the drug delivery means such that the rate at which the drug is delivered thereby during the inspiratorial flow is dependant upon the temporal value of that inspiratory flow.

In one embodiment, the control means are adapted to selectively operate the drug delivery means during said inspiratory flow at a frequency which is a function of the temporal value of the inspiratory flow.

In another embodiment, the control means are adapted to selectively operate the drug delivery means during the inspiratory flow such that the amount of drug delivered at each operation is a function of the temporal value of the inspiratory flow.

The following description refers in more detail to the various features of the respiratorial drug administration device of the present invention. To facilitate an understanding of the invention, reference is made in the description to the accompanying drawings where different embodiments of the respiratorial drug administration device are illustrated. It is to be understood that the respiratorial drug administration device of the present invention is not limited to the embodiments as illustrated in the drawings.

In the drawings :
- Figure 1 is a perspective view of one embodiment of a respiratorial drug administration device according to the present invention;
- Figure 2 is a perspective view of another embodiment of the respiratorial drug administration device according to the invention;
- Figure 3 is another perspective view of the drug administration device of figure 1;
- Figure 4 is a schematic block diagram of the drug administration device of figure 1;
- Figures 5 is a graphical representation of the inspiratory flow of a patient when using the drug administration device of figure 1;
- Figure 6 is a graphical representation of the frequency of operation of the drug delivery means of the drug administration device of figure 1;
- Figure 7 is a graphical representation of the amplitude of pulses applied to the drug propulsion means of the drug administration device of figure 1;
- Figure 8 is a schematic view of one embodiment of an active unit dose device of the respiratorial drug administration device of figure 1;
- Figure 9 is a schematic view of part of the active unit dose device of figure 7;
- Figure 10 is a schematic view of a variant of the active unit dose device of figure 7;
- Figure 11 is a schematic diagram of a chip section for use in the respiratorial drug administration device of figure 1;
- Figure 12 is a graphical representation of the output signals of the chip section shown in figure 11 and the corresponding frequency of the voltage pulses applied to the active unit dose device of figure 8;
- Figure 13 is a schematic diagram of a CMOS chip for use in the respiratorial drug administration device of figure 1, and
- Figure 14 is a schematic block diagram of another embodiment of a respiratorial drug administration device according to the invention.

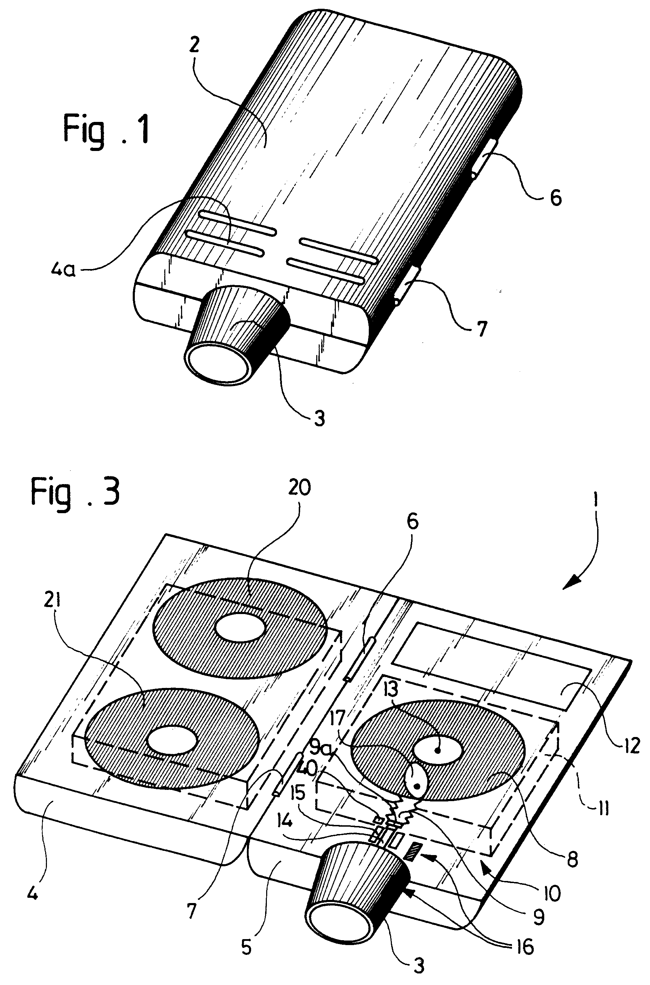

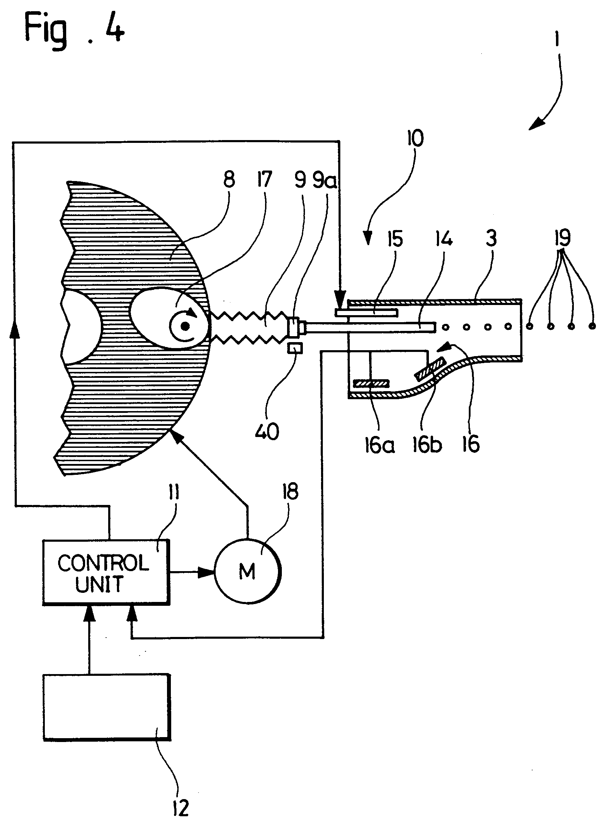

Referring now to figures 1 and 3, there is shown a schematic representation, respectively in a closed position and an open position, of one embodiment of a respiratorial drug administration device 1 according to the present invention. The respiratorial drug administration device 1 comprises a housing 2 and a mouthpiece 3 in communication with the interior of the housing 2. For ease of utilisation, the housing 2 may consist of two or more separable parts 4 and 5, interconnected by hinges 6 and 7 or by other appropriate releasable securing means.

The lower housing part 5 has mounted therein a magazine 8 containing one or more drug reservoirs 9 (only one of which is shown), a drug delivery system 10, electronic control circuitry 11, and a portable power source 12, such as a lithium battery. The magazine 8 is rotatably mounted in the lower housing part 5 about an axis 13. The drug reservoirs, which may each contain a unit dose such as 30 to 50 µl or another suitable small amount of drug in solution or suspension, are radially supported on or in the magazine 8 so that, upon rotation of the magazine 8 about the axis 13, each reservoir may be placed, in turn, in a delivery position as shown by placement of the drug reservoir 9 represented in figure 3. The magazine may be caused to rotate about the axis 13 by a stepper motor 17 controlled by the electronic control circuitry 11.

The upper housing portion may conveniently contain additional magazines 20 and 21, each containing one or more drug reservoirs, which may be mounted for rotation about the axis 13 by the patient at a later time.

The housing part 4 preferably includes suitable venting holes 4a and air ducting 4b, located under the magazines 20 and 21, which allows for a low resistance inspiratory airpath to the mouthpiece from where on the drug to be administered will carried with the inspiration. The ducting 4b also allows for locating diagnostic sensors and trapping expired air for analysis by said sensors as will described later.

When the drug reservoir 9 is in the delivery position, the drug delivery system 10 is adapted to cause the therapeutic drug contained in the reservoir 9 to be supplied into the mouthpiece 3, so that it may be taken up by the inspiratory flow of a patient. To that end, the drug delivery system 10 comprises a drug supply passage 14, one of more electroresistive elements 15, an air flow sensor 16 and a reservoir pressurising device 17.

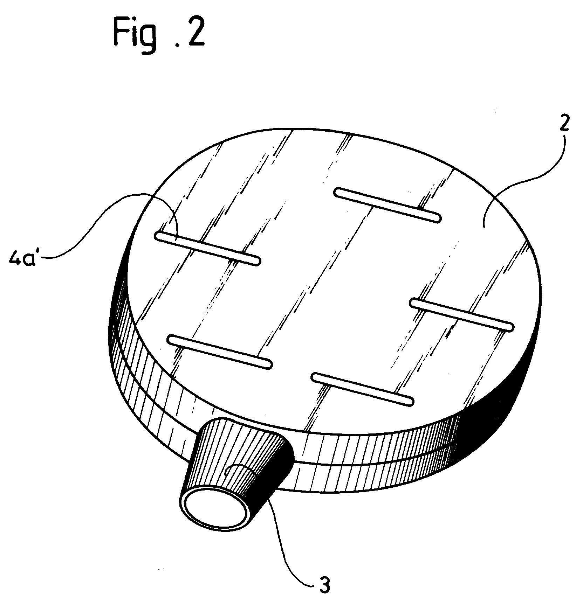

Figure 2 shows a variant of the device shown in figure 1, this variant having a circular, disk-shaped housing 2' connected to a mouthpiece 3' representing a more compact embodiment with only one disposable magazine.

Conveniently, the drug supply passage 14 may be constituted by a tube made of glass or like material such as a ceramic or alumina having an outlet of defined diameter, such as 10 µm or less, and may be mounted to the drug reservoir by a holder 9a. The reservoir pressurising device 17 is, in the example shown in figures 2 and 3, constituted by an actuator such as an off-center cam which is spring-biased so as to resiliently bear against the drug reservoir 17 during the delivery of the drug contained therein. The drug reservoir 9 is preferably a collapsible container. There is thus provided an arrangement whereby the drug in the reservoir 9 is supplied to the drug supply passage under a slight pressure which is maintained whilst the drug is gradually emptied from the reservoir 9.

The electroresistive elements 15, which are mounted onto the outer surface of the glass tube 14, may be constituted by piezoelectric devices. The electronic control circuit 11 is connected to the electroresistive elements 15 and is adapted to provide them with a series of voltage pulses. When, under the control of the electronic control circuit 11, one of these voltage pulses is applied to one of the electroresistive elements 15, the element undergoes a deformation. This deformation is transmitted to a portion of the glass tube 14, whereby a pressure pulse is applied to the drug therein. This pressure pulse acts to drive the drug in the glass tube 14 downstream from the electroresistive elements towards the outlet of the glass tube 14 and ejects the drug in the form of a microdroplet.

Each collapsible reservoir 9 may conveniently have associated therewith a number of elements of the drug delivery system 10. In this way, a number of active unit dose devices, each constituted, for example, by an arrangement comprising a collapsible reservoir, a tube and a piezoelectric actuator, may be manufactured as single units, preferably disposable. These units may then be mounted in a disposable magazine, which by being moved into the correct position, allows each active unit dose device to deliver its dose on request.

The disposable magazine can be an injection moulded wheel that carries a number of such active unit dose devices, appropriately held in position theraround, or the magazine can be formed to carry a blister tape, the blisters forming the collapsible reservoir on which the tube and piezoelectric actuator are appropriately attached.

It will also be appreciated that there exists a variety of actuation or motor possibilities to move a disposable magazine containing a number of the said disposable active unit dose devices in a rotational way such as to present one disposable active unit dose device after the other one in front of a mouthpiece or nosepiece for actuation and drug delivery by said disposable active unit dose device.

In a preferred embodiment the stepping motor, dedicated controller, cam shaft and disposable magazine axis are mounted on the same printed circuit board forming a preassembled and tested subsystem, thus limiting the total amount of components to be assembled in the final automatic assembly. Each disposable magazine is preferably also a prefiled and sealed subsystem, snap fitted to its axis on the printed circuit board.

In the preferred embodiment and for high precision drug delivery configurations, the individual disposable active unit dose devices will have been precision filled with a microfluid dosage control using piezoelectric differential pressure sensors with a measuring range of equal or better than 50 µl/s and a resolution of equal or better than 0,5 µl/s for a 1 second filling cycle time on an automatic reservoir filling and sealing line.

It will be appreciated that the arrangement shown in figures 1 to 4 can be realised in a variety of alternative embodiments. For example, a drug administration device according to the invention may be implemented as a pen-type, unit-dose rechargeable drug administration device, which can contain no or only a few spare disposable active unit dose devices, and may be fitted with a snap-in type positioning mechanism for locating the active unit dose devices. Simple electronic circuitry may also be used, since the drive and the drive control for the rotary motion disposable magazine may be replaced with a linear drive magazine in this case.

In one embodiment, each active unit dose device uses an injector tube substantially made of alumina, due to its excellent frequency matching with piezoelectric devices and its wide spread use as a substrate for printed circuits.

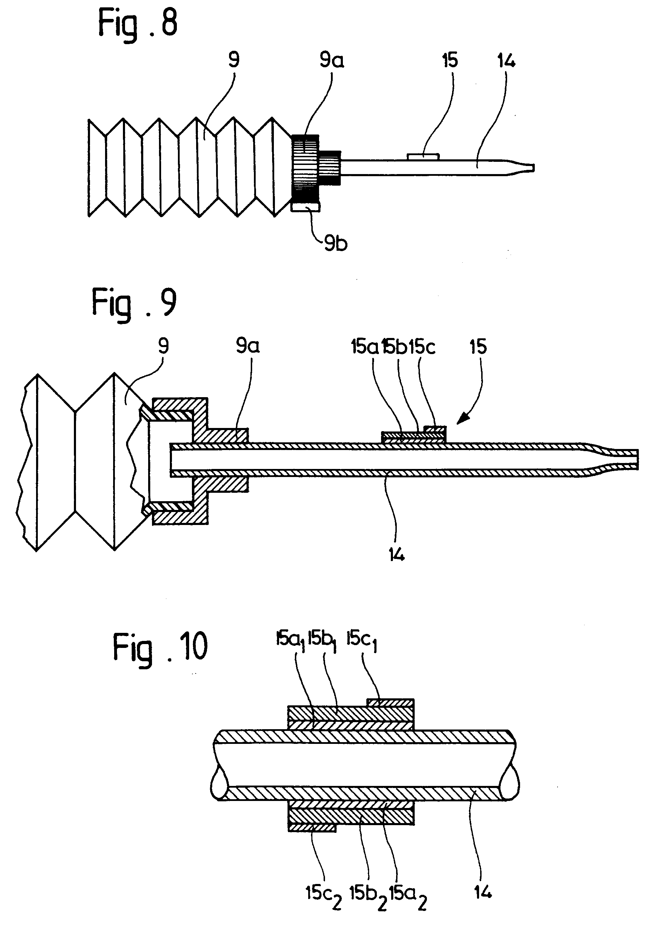

Extruded, annular piezoelectric actuators suitable for use in the invention may be formed in a number of ways. However, in a preferred embodiment, the piezoelectric actuators are deposited on the surface of the injector tube by a widely used screen-printing method, in order to reduce cost and increase the operational flexibility of the resultant device. This fabrication method, using ink containing PZT (lead zirconate titanate) material, as well as the ensuling firing or heat curing process, are mass production processes, leading to low cost production. This fabrication method also allows the deposition of one or more piezoelectric actuators, each consisting of the actuator PZT material and electrodes made of a suitable metal, on the outer surface of the injector tube and/or on other parts of the active unit dose device in a way to produce appropriately sized microdroplets and at a frequency suitable for inspiratorial drug delivery. In that sense, the active unit dose device actually represents a low cost drug delivery pump for kinetic energy applications, such as respiratorial therapy as described in the present application, as well as an add-on device to parenteral or eteral infusion systems where the microdroplets are delivered into the main delivery system instead of the inspiration air stream. Figures 9 and 10 show two embodiments of the active unit dose device in which the piezoelectric actuator 15 is shown to comprise at least one layer 15a of actuator PZT material and at least one electrode 15b on each such PZT layer.

In one embodiment, the injector tube 14 is at least partially produced by ceramic injection moulding, a process which provides a high quality, smooth and chemically amorphous interior surface as well as very low production costs in large quantities. The moulding process also allows for shaping of the tube interior for optimal flow and microdroplet production.

The disposable character of such an active unit dose device allows for a cost effective design of the key components of the respiratorial drug administration device of the present invention.

It should be noted that for the purpose of delivering drug solutions as well as suspensions, the injector tube holder 9a may required a modification of its properties. Figure 8 shows a side-view of the holder 9a and its relation to the reservoir 9 and the injector tube 14 in more detail. For drug delivery as a solution, the holder 9a, like the reservoir 9 itself, may preferably be an injection moulded part made of a suitable medical grade polymer. For suspensions, however, the holder 9a may be made as a ceramic injection moulded component, allowing the piezoelectric actuator frequency in effect to function as a resonant stirring agent. Preferably, an additional piezoelectric actuator 9b acting as such a resonant stirring agent is fixed to the holder 9a or to the reservoir 9. Other medical grade injection moulded materials, such as 316 L stainless steel, may also be used for the purpose of the invention.

In the case of emulsions, for example oil in water emulsions, a short heating pulse may be required to be applied to the drug to be administered in some cases for viscosity control. To this effect, a heating resistor 40 may be mounted on the injector tube holder 9a or elsewhere in the active unit dose device. In this case, the holder 9a may preferably be made of injection moulded ceramic material. Connections to the heating resistor may be made via spring-loaded connecting elements (not shown) or other suitable connection means.

Figures 9 and 10 show two embodiments of the active unit dose device in which one or more such heating elements or resistors 15c are mounted on corresponding electrodes 15b.

Figure 4 represents a schematic block diagram of the main elements of the respiratorial drug delivery device 1 shown in figures 1 and 3. In this embodiment, the air flow sensor 16 comprises two pressure sensing elements 16a and 16b separated from each other by a predetermined distance. In a known way, each pressure sensing element 16a and 16b is adapted to generate an electrical signal corresponding to the atmospheric pressure exerted on its surface. These electrical signals are compared by the electronic control circuit 11 so as to calculate the pressure difference detected between the two pressure sensing elements. The electronic control circuit 11 comprises a memory device (not shown) in which a previously determined correspondence between pressure drop and airflow for the respiratorial drug delivery device has been stored. The electronic control circuit 11 thus uses this memory device as a look-up table to determine the inspiratorial airflow of a patient based upon the pressure difference detected between the two pressure sensing elements 16a and 16b.

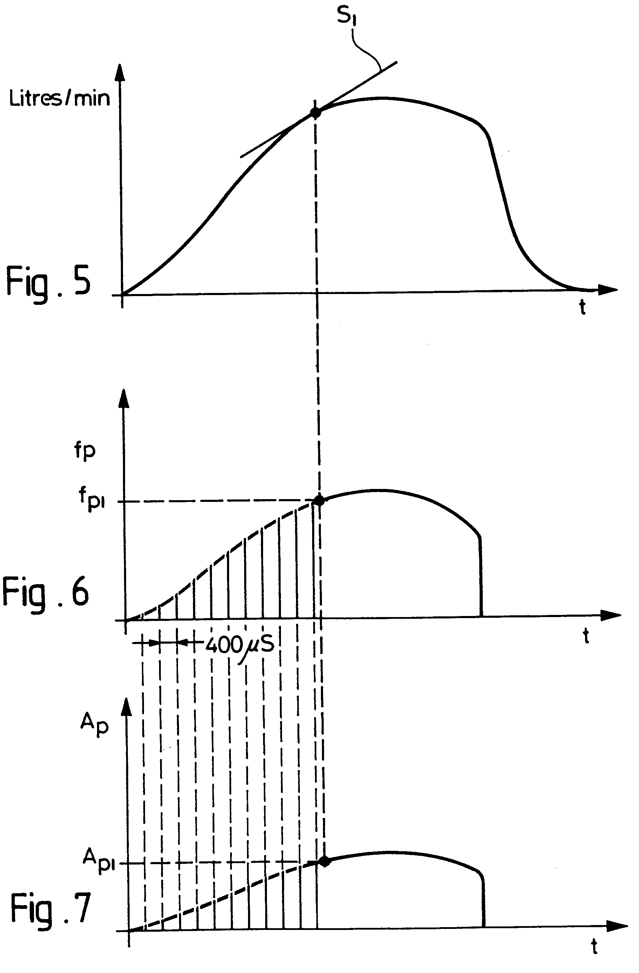

Figure 5 shows an example of the airflow detected by the respiratorial drug delivery device during a typical inspiration by a patient. At the start of the inspiration period, the airflow is 0 litres/min. The airflow then increases in a non-linear manner to a peak value, before decreasing again to 0 litres/min, at which time the patient exhales. In order that the microdroplets 19 ejected from output of the glass tube 14 are efficiently delivered to the patient, the electronic control circuit 11 is adapted to send voltage pulses to the piezoelectric element 15 at a frequency which is a function of the temporal value of the patient's inspiratorial flow, as detected by the airflow detector 16.

Accordingly, microdroplets of therapeutic drug are ejected from the outlet of the glass tube 14 as they are able to be taken up by the airflow of the patient. Variations in the volume of air per minute which is inspired by the patient are transformed into corresponding variations in the amount of drug ejected into the mouthpiece 3, so that the efficiency with which the drug is delivered to the patient is optimised.

In order that the dosage of the medication supplied to the patient may be controlled, the electronic control circuit 11 may be adapted to supply only a predetermined number of pulses to the electroresistive elements 15. If the inspirational flow of the patient is insufficient for the entire dosage of drug to be delivered during that inspirational flow, the patient need simply inspire again for the delivery of the dosage to be completed.

One advantage of a disposable active unit dose device as described above is the precise dosage in the form of a string of microdroplets via drop by drop actuation, with each microdroplet having the same spherical shape and volume as determined by the nozzle. For example, it has been determined that a 10 µm diameter nozzle leads approximately to a 5 pl individual spherical microdroplet. This is at the same time the resolution with which a 50 µl dose can, for example, be metered, delivered and inhaled, since the actuation can be electronically stopped at the count of one microdroplet if so programmed.

Moreover, prior art devices deliver a therapeutic drug to a patient by producing an atomised cloud containing this drug into the mouth of the patient. Drug is therefore delivered in random directions inside the patients mouth, only a portion of the delivered drug actually being taken up in the patient's airflow. According to the present invention, the microdroplets of therapeutic drug are ejected along a substantially uniform path into the inspiratory flow of the patient and are thus delivered directly into this inspiratorial flow, in this way avoiding inefficient delivery and requiring less total drug to be delivered.

A further advantage of the present invention is the low speed and kinetic energy of the individual microdroplet in the delivery string at the outlet of the active unit dose device which assures that they will be easily synchronised with the breath, carried on with the inspiratory, mostly laminar air flow. This also largely avoids the problem of significant oropharyngal deposition through impactation and subsequent swallowing which is posed by known respiratorial drug administration devices and the consequent problem of an often large and inconsistent difference between metered, delivered and inhaled doses. Such a string of microdroplets adapts to the inspiratory flow in such a way as to largely distribute its dosage volume proportionally throughout the trachea, down to the deep lung. Thus, the continuous curve mirroring and the rule processing of the control circuit 11, adapt the frequency of operation of the drug propulsion means 13 to the inhalation of the patient.

A further advantage is the fine granular adaptability of the microdroplet size and the spacing between microdroplets to the therapy and of the microdroplet delivery frequency to the inspiratory flow of the user until the targeted dose is completely delivered or the inspiration is terminated, allowing for maximum of consistency over the whole range of the patient's inhalation conditions.

A further advantage is the low production cost of the disposable active unit dose device through the use of a few, simple components which can be made and assembled with mass production means and configured in various styles ranging from a pen-type device to a single magazine disk-type device and to a multi magazine device as shown in figures 1 to 4, or even to an active add-on to a drug containing flask for various infusion applications.

A further advantage is the adaptability to drug delivery in various formulations, through the choice of materials with appropriate frequency response or through heating elements integrated into the active unit dose device.

In order to optimise the response time with which the frequency of operation of the electroresistive elements 15 tracks that of the detected inspiratory flow, the electronic control circuit 11 is preferably configured as a controller responding at least in part to the derivative of the inspiratorial flow. In this way, the temporal change in inspiratorial flow (i.e. the slope S₁ of the curve shown in figure 4) is used as a factor to determine the frequency fₚ₁ of operation shown in figure 5 of the electroresistive elements 15. Any change in inspiratorial flow is thus quickly detected and converted into an corresponding change in the frequency of the voltage pulses sent to the piezoelectric elements 15.

In another embodiment, and as shown in figure 6, the electronic control circuit 11 may be adapted to send voltage pulses to the piezoelectric element 15 at a constant frequency but at an amplitude Aₚ₁ which is a function of the temporal value of the patient's inspiratorial flow. In this way, the magnitude of the pressure pulse applied to the drug in the glass tube 14 is varied so as that the amount of drug ejected from the glass tube outlet, following the application of voltage pulse, is a function of the temporal value of the patient's inspiratorial flow.

The present invention may also be used as a diagnostic tool in order to determine the total quantity of drug required to be delivered to the patient at any one time for effective treatment of his or her condition. The dosage of the medication administered to a single patient during the course of a therapy can therefore by altered, or alternatively, the same delivery device may be used to deliver varying dosages of the same or different drugs to a several patients. To this effect, the respiratorial drug administration device may also include means for analysing one or more characteristics of an expiratory flow of the patient, and means for regulating the total amount of drug delivered by the drug delivery means as a function of this or these expiratory flow characteristics.

For example, the expiratorial flow of a patient may be used by the respiratorial drug administration device. In that regard, the forced expiratory volume per second and the peak expiratory flow rate, for example, can be measured and used to diagnose the existence of medical conditions or to ascertain the efficiency of a drug therapy program. The expiratory flow rate can be used, for example, in the case of patients suffering from asthma, to determine the appropriate dosage of medication which should be administered to the patient.

In one embodiment, the respiratorial drug administration device 1 is equipped with an expiratorial flow sensor for measuring the airflow when a patient exhales into the mouthpiece 3. Conveniently, this flow sensor may be constituted by the above-described airflow sensor 16, which may thus be used to measure both the inspiratorial and expiratorial flow of a patient. Whether the patient is inhaling or exhaling may easily be determined by the electronic control circuit 11, as the pressure difference detected between the two sensing elements 16a and 16b will be positive in one case and negative in the other. Other techniques for flow measurement like thermal absorption or analysis of the Seebeck effect, optical fibre based and other optical sensors can also be used in conjunction with the curve mirroring technique as well as combinations between flow, pressure, temperature and other physiological measurements.

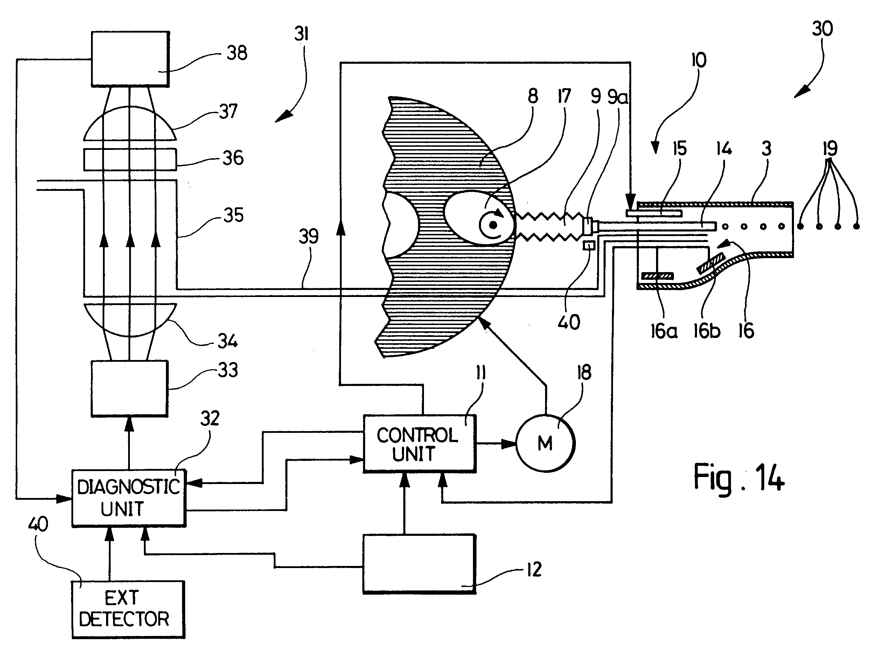

Alternatively, the characteristics measured may be the constituent parts of the air exhaled by the patient may analysed in order to determine the appropriate dosage of the therapeutic drug which should be administered to the patient. Figure 14 shows one such example, in which the acetone content of a patient's breath is analysed in order to determine the dosage of appropriate medication to be delivered to the patient.

The respiratorial drug administration device 30 shown in this figure includes all components of the drug administration device 1 shown in figure 3. The operation of these components in identical to that described in relation to figure 3. In addition, the respiratorial drug administration device 30 comprises, in this example, an acetone detector 31 and diagnostic circuitry 32 for regulating the total amount of drug delivered by the drug delivery system so as a function of this expiratory flow characteristics. The acetone

When in use, the patient firstly exhales into the mouthpiece 3. Air from the patient's expiratorial flow is delivered into the acetone detector 31 and an electrical signal representative of the level of acetone present in the patient's breath at the time of measurement is then supplied to the diagnostic circuitry 32 for analysis. Based upon this electrical signal, the diagnostic circuit 32 may be adapted to calculate a corresponding dosage of the medication to be delivered to the patient. For example, the number of voltage pulses required to deliver a corresponding number of microdroplets so as to administer these microdroplets calculated dosage may then be evaluated by the diagnostic circuit 32 and then sent to the control circuit 11. This latter may then deliver the microdroplets of drug to the patient, in the above-described manner, until this predetermined number of microdroplets is obtained.

Methods of using breath acetone as an indicator for blood glucose due to the close relationship between blood glucose, serum acetone and alveolar acetone and devices using such techniques are already known, as is the use of other parameters such as H₂ for persons on a diet or ethanol for persons having consumed alcohol.

These devices are complex and have not been adapted to respiratory therapy devices. A problem with such devices is to make sure that only alveolar or mostly alveolar air is measured and correlated to the therapy. For example, for diabetic patients this can be achieved according to the present invention and in particular its curve mirroring technique, by following the expiratory airflow, its temperature and measuring the acetone content at the moment of highest breath temperature and integrating the portion measured just before the expiration air flow arrives at its end, using air trapped in the air ducting section of the device according to the invention, appropriate sensors located in that section and appropriate closing flaps, which define and trap the volume to be measured and/or analysed.

It is generally known that when a person expires, he or she expires first a certain amount of so-called dead volume of air, meaning air mostly resting in the lung between flat respirations, some fresh air which after inspiration has penetrated into deeper layers of the lung and finally alveolar air coming from the 23rd generation of the lung, which contains a large number of volatile and other components that can be used for diagnostic purposes. During the process of expiration, the temperature of the expired air increases, generally from about 25°C to ≈ 34°C and stays constant around the last temperature value at the end of the expiration or when alveolar air is exhaled.

This alveolar air being the closest to the blood interface, it contains also the closest approximation of the correct concentration of various diagnostic components in the blood, for example, acetone for diabetics, H₂ for people on a diet, CO₂ corresponding to mixed venous CO₂, which again is an approximated measure of arterial CO₂, pH etc. If the concentration is very high, the volatile component can actually be smelled by other humans as is the case with diabetics in a severe condition where the smell of their breath resembles cider or apple juice.

The first step then is to measure the temperature of the expired air or actually to follow or mirror its rise, e.g. slope during the expiration process. It will be appreciated that measuring the exact temperature value, say with a precision of 0.1°C is relatively futile because every breath is different and every patient has his or her own "signature" in physical and electronic terms on this parameter. The important feature is to capture the moment as of which the said temperature stabilises which means that as of that moment alveolar air is being analysed and that the measured concentration is relevant for diagnostic purposes. The next step is to integrate the measured values until the expiratory flow signal points to the end of the exhaling cycle and to consider for diagnostic purposes only an integral of all values measured from, say 200 ms or another small number of ms before the drop-off of the expiration flow curve.

This function can be realised for acetone and H₂ content, both being combustible gases, using an acetone detector 31 constituted by a semiconductor catalytic gas sensor, or resistive sensors, with an integrated temperature sensor, both for device selectivity and temperature curve mirroring. State of the art semiconductor sensors, used for well-known ethanol measuring applications (alcohol sensor), in the case of acetone, for example, can have a sensitivity of ≈ ≤ 1 ppm. It will be appreciated that this allows, in conjunction with the curve mirroring technique to supervise the blood glucose content of a patient frequently, non-invasively by repeated expiration into the device, thus supplementing the known invasive method.

In effect, the acetone concentration reading will be subjected to a tight interpretation control range related to the direct blood glucose measurement and inside this control range it can make invasive blood glucose measurement redundant and outside his range it will point to the necessity of confirming the acetone base reading with an actual blood glucose sensor reading.

Curve mirroring and rule based circuits such as described below improve the simplicity and reliability of the device, as well as enhancing the possibility to deliver small, very precise doses frequently.

It will also be appreciated that the invention improves the quality of life not only for asthmatics but also for diabetics since together with the highly precise respirational drug administration system proposed, it forms an ideal tool in both cases for less invasive therapy and a better base line control through the possibility of more frequent, non-invasive readings and the equally non-invasive possibility of frequent, precise small doses of medication and the possibility to establish and monitor daily delivery patterns with a hitherto unknown resolution in time and dosage.

In addition, the respiratorial drug delivery device 30 may comprise an interface 40 between an external sensor and the diagnostic circuitry 32. Such an interface may be used, for example, in the case of where external sensors for measuring blood glucose levels, or other sensors for obtaining information which is not conveniently available through the analysis of the patient's expiratorial flow, are required. This information may be used separately from or in combination with internal sensors, such as the breath acetone sensor 31, in order to determine the dosage of a therapeutic drug required to be delivered to a patient.

The control circuitry 31 and/or diagnostic circuitry 32 preferably comprises one or more fuzzy logic circuit elements. The feature of fuzzy logic theory is that uncertain linguistic information can be handled qualitatively using membership functions. Fuzzy logic circuitry is thus ideally suited to the diagnosis of medical conditions which only require qualitative results (i.e. good glucose level control, poor glucose level control, medium glucose level control) to be obtained from such diagnosis. Advantageously, such circuitry may be realised without the complexity, high calculating power and high-power consumption necessitated by classic digital and analogue circuitry, thus reducing the cost and power consumption of the respiratorial drug delivery device according to the invention, as well as enhancing its portability.

One particularity of the invention and the disposable active unit dose system is the high resolution with which the delivery process can be controlled, as described above, actually on the count of one microdroplet. This is possible with the use of an inhalation flow sensor, conventional microprocessors and drive circuit. However, a microdroplet of 5 pl represents a resolution or precision of 10 ppm of a total dose of 50 µl. This precision and the amount of inhalation flow and delivery control data processing is, perhaps with the exception of extremely expensive drugs generally not needed or useful.

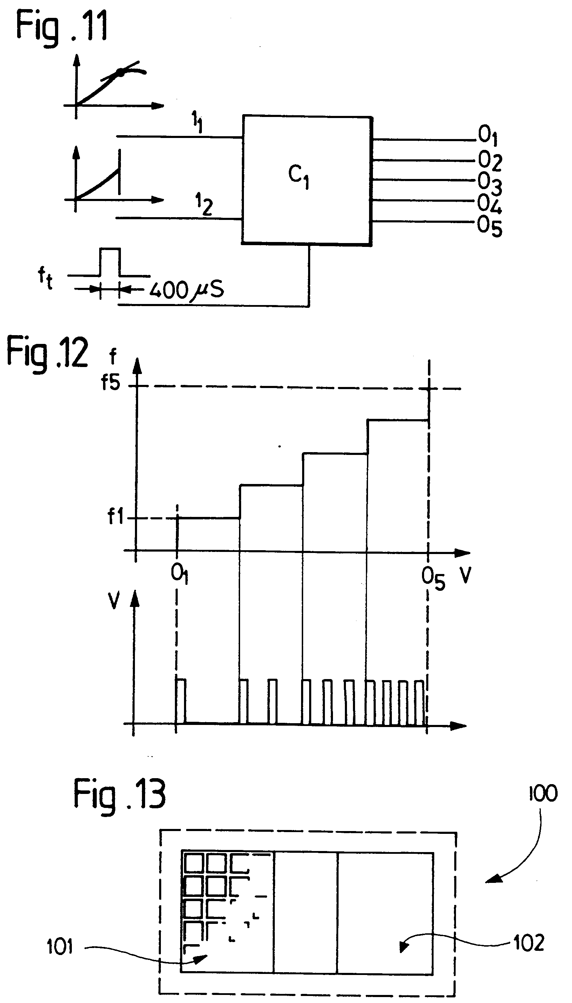

In a preferred embodiment of the invention shown in figures 11 to 13, the control circuit 11 of figure 4 and the combination of the control circuit 11 and diagnostic circuit 32 of figure 15 may be realised by a CMOS chip 100 which comprises two sections, one section 101 containing a very low power analog CMOS fuzzy rule processing module for inhalation curve mirroring, for reservoir compression and positioning control, diagnostic sensor interface etc and one section 102 realised in high voltage low current CMOS (for example 40 to 80 V) for piezoelectric actuation with frequency curve mirroring according to the inhalation curve and other drive function rule processing.

The very low power analog CMOS fuzzy rule chip section 101 of the chip 100 consists of a number of analog CMOS rule circuits C₁ which for example each have two or more inputs and 5 or more outputs. Such a chip section C₁ can contain for example 70 such rule circuits C₁ on a 1 mm² chip surface. It processes the low curve as measured by the inspiration flow sensor 16 in time slices of ≥ 400 µs, as shown in figures 6 and 7, over an inspiration time duration of several seconds. The inputs I₁, I₂, mirror the inspiration flow curve by one input receiving the flow value directly and the other input by measuring the slope or change in inspiration flow curve compared to the last reading which was taken, for example 400 µs before, if this was the cycle time. As shown in figure 12, the outputs 0₁-0₅ follow a set of rules allowing to adjust the actuation frequency and/or amplitude, for example, in five steps each, in order to mirror the inspiration flow curve in the drug delivery actuation parameters followed by the high voltage CMOS section of the chip, i.e. the frequency of microdroplet generation, the distance in space and time of flight between microdroplets and the amplitude of the actuation voltage applied to the disposable active unit dose device.

It can be appreciated that such a solution enables to initiate and stop drug delivery at any point and at any time of the inspiration flow curve, to mirror and follow any inspiration flow curve, to work with a large variety of resolutions, to know exactly how many microdroplets have been delivered within the chosen resolution factor in % or ppm and how many might still need to be delivered to complete the full dose in case the patient had only a very short and flat inspiration. The freedom to follow any inspiration flow curve, to initiate the disposable active unit dose device at any time or point of the inspiration flow curve makes the respiratorial drug delivery device independent of the inhaling flow capacity of the patient and the freedom to chose any cut-off point, especially when the slope of the inspiration flow curve is falling, even steeply, avoids that the drug delivered by the respiratorial drug administration device is deposited into the buccal cavity or actually exhaled again. The administration of a respirable dose over the whole range of continuously patient inhalation conditions can be forgetted by combining the advantages of the disposable active unit dose device and the inspiration flow curve mirroring described above.

The combination for the disposable active unit dose device and the dedicated control system are an advantage, especially for very expensive systemic drugs or for frequent administration of low doses such as for the base line dosing of insulin for diabetics or for migraine treatment but also for the confidence of the patient.

It can be appreciated that the invention is not limited to the timing and input/output configurations described, but that the proposed chip structure and layout and the active unit dose device allow for adaptation to a variety of respiratorial therapy requirements.

Those skilled in the art will appreciate that there may be many variations and modifications of the configuration described herein which are within the scope of the present invention.

In that regard, the invention can also be used to provide respiratorial diagnostic means suitable for use as an interface with external diagnostic devices which are appropriately connected to the control circuitry 11 of figure 4 and having dedicated evaluation and, preferably, display means. It is a particularity of the invention that the respiratorial drug delivery means and the respiratorial diagnostic means can function for a variety of therapy factors to be diagnosed, and can be used independently as stand-alone means, complimentary to other drug delivery systems, or as components of a modular respiratory drug delivery device such as described hereabove.

## Claims

1. Device for the respiratorial administration of a drug to a patient, comprising :
- a flow sensor (16) for detecting an inspiratory flow of said patient;
- drug delivery means (10) for delivering an amount of said drug;
- control means (11) for causing the operation said drug delivery means, characterised in that said control means (11) are adapted to selectively operate said drug delivery means (10) such that the rate at which said drug is delivered during said inspiratorial flow is dependent upon the temporal value of said inspiratory flow.

2. Device according to claim 1, characterised in that said control means (11) are adapted to selectively operate said drug delivery means (10) during said inspiratory flow at a frequency (Fₚ₁) which is a function of the temporal value of said inspiratory flow.

3. Device according to claim 1, characterised in that said control means (11) are adapted to selectively operate said drug delivery means during said inspiratory flow such that said amount of drug released at each said operation is a function of the temporal value of said inspiratory flow.

4. Device according to any of the preceding claims, characterised in that said drug delivery means (10) comprises :
- a drug supply passage (14) having an inlet and an outlet;
- drug propulsion means (15) for producing a pressure pulse in the drug in said drug supply passage (14) to cause a predetermined amount of drug to be displaced towards and ejected from said outlet in the form of a microdroplet.

5. Device according to claim 4, characterised in that said drug supply passage (14) and said drug propulsion means (15) mutually act to eject said microdroplets along a substantially uniform path into said inspiratory flow.

6. Device according to any one of the preceding claims, characterised in that it further comprises :
- a drug reservoir (9) in fluid communication with the inlet of said drug supply passage (14); and
- pressurising means (17) for pressurising the drug in said drug reservoir (9).

7. Device according to claim 4, characterised in that said drug reservoir (9) is constituted by a collapsible recipient, and in that said pressurising means (17) comprises an actuator for bearing against and, as said drug is delivered, collapsing said drug reservoir.

8. Device according to any one of the preceding claims, characterised in that it further comprises :
- means (31) for analysing one or more characteristics of an expiratory flow of said patient, and
- means (32) for regulating the total amount of drug delivered by said drug delivery means as a function of said expiratory flow characteristic.

9. Device according to claim 8, characterised in that said regulating means (32) comprise one or more fuzzy logic circuit elements.

10. Device according to any one of claims 6 or 7, characterised in that said drug supply passage (14), said drug reservoir (9) and said drug propulsion means (15) at least partly form a single-use active unit dose device.

11. Device according to claim 10, characterised in that said drug supply passage (14) is constituted by a tube mounted to said drug reservoir (9) by a holder (9a).

12. Device according to claim 11, characterised in that said tube (14) is substantially made of alumina, ceramics or stainless steel.

13. Device according to either of claims 11 or 12, characterised in that said drug propulsion means (15) comprise at least one piezoelectric device deposited on the outer surface of said tube (14).

14. Device according to claim 13, characterised in that said drug propulsion means (15) comprises at least one layer (15a) containing PZT material.

15. Device according to any one of claims 10 to 14, characterised in that it further comprises at least one heating element (40; 15c) mounted on said active unit dose device.

16. Active unit dose device for a respiratorial drug administration device, comprising :
- a collapsible drug reservoir (9) containing a unit dose of a drug;
- a drug supply tube (14) in fluid communication with said drug reservoir (i), and
- drug propulsion means (15) for producing a pressure pulse in the drug in said tube (14) to cause a predetermined amount of drug to be displaced towards and ejected from said outlet in the form of a microdroplet.

17. Active unit dose device according to claim 16, characterised in that said tube (14) is mounted to said drug reservoir (9) by a holder (9a).

18. Active unit dose device according to either of claims 16 or 17, characterised in that said tube (14) substantially made from alumina, ceramics or stainless steel.

19. Active unit dose device according to any one of claims 16 to 18, characterised in that said drug propulsion means (15) comprise at least one piezoelectric device deposited on the outer surface of said tube (14).

20. Active unit dose device according to any one of claims 17 to 19, characterised in that said holder (9a) is substantially made from a polymer.

21. Active unit dose device according to any one of claims 17 to 20, characterised in that it further comprises a piezoelectric actuator (9b) acting as a resonant stirring agent which is fixed to the holder (9a) or to the reservoir (9).
